# EUROPEAN PATENT SPECIFICATION

(11) **EP 1 067 139 B1**
(45) Date of publication and mention of the grant of the patent: **16.08.2006**
(21) Application number: 99112987.5
(22) Date of filing: 06.07.1999
(51) Int. Cl.: C07K 14/43, C12N 15/29, G01N 33/50, A61K 39/35, A61K 38/10

(54) **Cyclophilin identified as an allergen**
Cyclophilin als Allergen identifiziert
La cyclophiline identifiée comme allergène

(43) Date of publication of application: 10.01.2001
(73) Proprietor: SCAP foundation, 2011 KS Haarlem (NL)
(72) Inventor: Cadot, Pascal Gilbert, Dr., 3360 Bierbeek (BE); Ceuppens, Jan Louis, 3001 Heverlee (BE)
(74) Representative: Sternagel, Fleischer, Godemeyer & Partner

(56) References cited:
- HORNER W.E ET AL., : "Identification of the allergen Psi c 2 from the basidiomycete Psilocybe cubensis as a fungal cyclophilin" INTERNATIONAL ARCHIVES OF ALLERGY AND IMMUNOLOGY, vol. 107, no. 1-3, 1995, pages 298-230, XP002123481
- GASSER C.S. ET AL., : "Structure and expression of cytosolic cyclophilin/peptidyl-prolyl cis-trans isomerase of higher plants and production of active tomato cyclophilin in Escherichia coli" PROC. NATL. ACAD. SCI. U.S.A., vol. 87, 1990, page 9519-9523 XP002123482
- DATABASE EMBL NUCLEOTIDE AND PROTEIN SEQUENCES,1 November 1996 (1996-11-01), XP002123483 HINXTON, GB
- DATABASE EMBL NUCLEOTIDE AND PROTEIN SEQUENCES,15 July 1998 (1998-07-15), XP002123484 HINXTON, GB
- DATABASE EMBL NUCLEOTIDE AND PROTEIN SEQUENCES,1 February 1997 (1997-02-01), XP002123485 HINXTON, GB
- DATABASE EMBL NUCLEOTIDE AND PROTEIN SEQUENCES,1 February 1994 (1994-02-01), XP002123486 HINXTON, GB
- SAITO T. ET AL., : "Cloning and sequence analysis of genes for cyclophilin from Arabidopsis thaliana" PLANT CELL PHYSIOL., vol. 36, 1995, page 377-382 XP002123487
- LIPPUNER V. ET AL., : "Cloning and characterization of chloroplast and cytosolic forms of cyclophilin from Arabidopsis thaliana" THE JOURNAL OF BIOLOGICAL CHEMISTRY, vol. 269, no. 11, 1994, pages 7863-7868, XP002123488
- LUAN S. ET AL., : "pCyP B: a chloroplast-localized, heat shock-responsive cyclophilin from Fava bean" THE PLANT CELL, vol. 6, no. 1994, pages 885-892, XP002123489
- TAKAHASHI N ET AL: "PEPTIDYL-PROLYL CIS-TRANS ISOMERASE IS THE CYCLOSPORIN A-BINDING PROTEIN CYCLOPHILIN" NATURE,GB,MACMILLAN JOURNALS LTD. LONDON, vol. 337, no. 6206, 1989, page 473-475 XP000082642 ISSN: 0028-0836
- BREITENEDER H ET AL: "THE GENE CODING FOR THE MAJOR BIRCH POLLEN ALLERGEN BETVL, IS HIGHLY HOMOLOGOUS TO A PEA DISEASE RESISTANCE RESPONSE GENE" EMBO JOURNAL,GB,OXFORD UNIVERSITY PRESS, SURREY, vol. 8, no. 7, 1989, page 1935-1938 XP000050673 ISSN: 0261-4189
- CADOT P ET AL., : "Influence of the pH of the extraction medium on the composition of birch (Betula verrucosa) pollen extracts" ALLERGY, vol. 50, 1995, pages 431-437, XP002123490
- APPENZELLER U ET AL., : "IgE-mediated reactions to autoantigens in allergic diseases" ALLERGY AND IMMUNOLOGY, vol. 118, no. 2-4, April 1999 (1999-04), pages 193-196, XP002123491
- LINDBORG M ET AL., : "Selective cloning of allergens from the skin colonizing yeast Malassezia furfur by phage surface display technology" JOURNAL OF INVESTIGATIVE DERMATOLOGY, vol. 113, no. 2, August 1999 (1999-08), pages 156-161, XP002123492

### SEQUENZPROTOKOLL

<110> SCAP foundation
<120> Cyclophilin as a new allergen
<130> 5673EPSeq
<140>
   <141>
<160> 1
<170> PatentIn ver. 2.1
<210> 1
   <211> 17
   <212> PRT
   <213> Betula verrucosa
<400> 1
   Asp Phe Thr Ala Gly Asn Gly Thr Gly Gly Glu Ser Ile Tyr Gly Ala
     1               5                  10                  15
   Lys

## Description

The present application refers to a protein with rotamase activity, which is a cyclophilin, for diagnostic and therapy of allergic diseases.

Many tree species (like birch - *Betula verrucosa)* produce large amounts of pollen that induce symptoms of Type I allergy (allergic rhinitis, conjunctivitis, asthma) on inhalation. Other botanically distinct tree species may contain allergens in their fruits, which cross-react with pollen allergens and therefore elicit food allergy on ingestion by pollen-allergic individuals.

Birch pollen is a major cause of allergy in the northern parts of Europe and America, as well as in some areas of Australia, with symptoms occurring in early spring. For this reason, it is of crucial importance to characterize and analyze the birch pollen allergens, proteins that are responsible for the clinical symptoms of birch pollen allergy.

It has been discovered, that the cross-reactivity observed among certain pollen species can be attributed to the structural and immunological similarity of relevant cross-reactive allergens. This finding implies that diagnosis and immunotherapy may be performed with a few cross-reactive marker allergens that harbor a large proportion of the cross-reactive epitopes.

Five birch pollen allergens have already been well-described so far. The major allergen is Bet v 1, a 17-kDa protein consisting of several isoallergens, and recognized by almost all birch pollen-allergic patients. The genes coding for Bet v 1 show similarity with genes involved in plant defense mechanisms (Breiteneder *et al.,* EMBO J. 1989; 8: 1935-1938). Proteins that resemble Bet v 1 are found in some fruits and vegetable and are responsible for food-pollen cross-allergies. The second well-known allergen is the birch pollen profilin, Bet v 2, that is involved too in cross-reactions between birch pollen and foodstuff (Ebner *et al.,* J Allergy Clin Immunol 1995; 95: 962-969). The two other allergens, namely Bet v 3 and Bet v 4, are minor allergens in that they are recognized by a small percentage of sera from birch pollen allergic patients. Both have been demonstrated to be calcium-binding proteins (Seiberler *et al.,* EMBO J. 1994; 13: 3481-3486; Engel *et al.,* J Biol Chem 1997; 272: 28630-28637). Recently, a 35-kDa protein, related to isoflavone reductases, has been shown to bind IgE of birch pollen allergics (Vieths *et al.,* Scand J Immunol 1998; 47: 263-272). It could also be involved in pollen-food cross-allergies.

Further Cadot *et al,* described in Allergy 1995; 50: 431-437 that birch pollen extracts prepared at pH 7.5 or 8.5 under continuous pH control contained three previously unknown allergens with pI 9, 9.1 and 9.3, possibly isoforms of one protein. The molecular mass could not be determined because the patients sera that were positive for these proteins in IEF immunoblots did not detect any new band in SDS-PAGE immunoblots, which could be explained by the involvement for recognition of conformational epitopes only. Direct sequencing on a blotting membrane was unsuccessful, which was attributed to N-blockage.

Rotamase (= Peptidyl-prolyl cis-trans isomerase) catalyzes rotation of the peptide bond on the amino side of proline residues in a protein or peptide. Rotamase is effectively inhibited by Cyclosporin A, a cyclic undecapeptide, which has immunosuppressive effects (Takahashi *et al.,* Nature 1989; 337: 473-475 and Fischer *et al.,* Nature 1989; 337: 476-478). The later discovered protein cyclophilin has been identified to be identical to one type of rotamase.

Cyclophilins are identified and isolated from various species, and gene homologous are identified in mammalian as well as in *Drosophila* and Prokaryotes. Further Cyclophilins are found in different species of higher plants.

Gasser *et al.* described in Proc. Natl. Acad. Sci. 1990; 87: 9519-9523 that the DNA sequences of the cyclophilins of the different plant species have high homology to each other as well as to cyclophilins of other species, i.e. human. The open reading frames of the genes encode proteins of 171-172 amino acids. The cyclophilins of the different plant species have more than 75% amino acid identity and more than 85% amino acid conservation. Further it has been discovered, that the cyclophilin gene does not contain any intron in the coding sequence. Recombinant tomato cyclophilin is expressed in *E. coli* and identified by rotamase activity.

*Horner, W.E., et al.* describe in INT. ARCH. ALLERGY IMMUNOL., 1995; 107:298-300, a recombinant cyclophilin from *Psilocybe cubensis* having antigenic activity with serum from patients having respiratory allergies.

*Cadot, P., et al.* describe in ALLERGY, 53 (suppl. 43) : 25 (1998), allergens from birch pollen, particularly a protein with three isoforms, which was recognized from sera of birch pollen allergic patients as an antigen.

*Trandinh, C.C., et al.* disclose 1992 in the THE FASEB JOURNAL 6 : 3410-3420 several cyclophilins, wherein the sequences of several species are aligned and compared. On basis of this comparison the evolutionary relationship among these proteins is discussed.

It is an object of the present application to provide means or remedies for diagnosis and therapy of allergic diseases.

This object is attained by a protein which contains SEQ ID NO: 1 for diagnostic or therapeutical use.

Preferably the protein according to the invention has rotamase activity.

More preferred the protein according to the invention is a cyclophilin. Cyclophilins are very highly conserved proteins, particularly plant cyclophilins have high homology to each other. Since all the known cyclophilins possess the same enzyme activity and are inhibited by the same molecule (cyclosporin A), the 3D-structure of these proteins must be very well-conserved too. Lippuner *et al.* showed in J. Biol. Chem. 1994; 269: 7863-7868 that antiserum raised against cytosolic cyclophilin of mouse-ear cress demonstrated cross reactivity with a broad range of cyclophilins from other sources. These results imply, that as well the antigenicity of cyclophilins is very similar.

Never before a plant cyclophilin has been described to be an allergen. This is also the first time, that it is described in pollen. The present application shows that plant cyclophilin is an important new allergen and can be used for diagnosis and therapy of allergic diseases, like allergic rhinitis, allergic asthma, allergic conjunctivitis and pollen-associated food allergies. Screening by IEF immunoblots with 48 birch pollen-allergic sera showed up to 20.8% sera reacting with cyclophilin.

One preferred object of the present application is to provide means for *in vivo* and *in vitro* diagnosis and therapy of birch pollen allergy. Because of highly conserved structure and antigenicity among cyclophilins, the present application further provides means for the diagnosis and therapy of pollen allergy.

A further preferred object of the present application is to provide means for *in vivo* and *in vitro* diagnosis and therapy of pollen-associated food allergy.

Cyclophilins are known to be contained in a various number of plants, their presence being mentioned in almost all organs studied: roots, stems, buds, seeds, and anthers. For this reason, and because of the highly conserved structure and antigenicity among cyclophilins, and especially among plant cyclophilins, involvement of cyclophilins in food-pollen cross-allergies seems evident. Up to now cross-reactivity of food allergy has never been tested referring cyclophilins. In the present invention plant cyclophilins containing SEQ ID No. 1 are used for diagnosis or therapy of food-pollen cross-allergies. In the most preferred embodiment cyclophilins are used, which are isolated from that plant against which the food-allergic person shall be tested. Combinations of cyclophilins from different plants are especially preferred.

The protein of the present invention can be a native or recombinant protein of a prokaryont or an eukaryont, i.e. from bacterial, yeast, fungi, plant, insect or mammalian source. Preferred is a protein of any eukaryont, more preferred from higher plants (i.e. foxglove, rosy periwinkle, tomato, and onion). The most preferred protein is birch pollen cyclophilin.

Extraction of allergens is possible in any aqueous solution. Pure water as well as any aqueous buffer system can be used. Tree pollen allergens are low-molecular-weight proteins (or glycoproteins) that rapidly elute from pollen after contact with aqueous solutions. Hydration of the pollen transports the allergens from the inner side to the surface of the pollen grains. The different tree pollen allergens are easily extracted from pollen in large amounts after hydration. Methods of extraction of corresponding recombinant proteins from cells of any expression system are commonly known to skilled artisans.

In a preferred embodiment allergen extracts are prepared in phosphate buffer with a pH range between 6,5 to 8,5, more preferred between 7,5 and 8,5.

Protein extracts can be purified by any known protein purification method, i.e. filtration, hydrophilic or hydrophobic interaction chromatography, ion-exchange, molecular filter chromatography, precipitation strategies, or combinations thereof to obtain a protein according to the present invention.

Proteins according to the invention contain an amino acid sequence SEQ ID N0: 1, which is DFTAGNGTGGESIYGAK, SEQ ID N0: 1 can be flanked by a sequence or parts of a sequence of a cyclophilin, at one or both ends. In a most preferred embodiment SEQ ID NO: 1 is internal part of a cyclophilin.

The protein according to the invention shows in a SDS polyacrylamide gel a single band at about 18 kDa and in an immunoblot analysis after isoelectric focusing (IEF) can show several bands in the basic pI range, representing various isoforms of the protein.

Further preferably the protein possesses a peptidyl-prolyl cis-trans isomerase (rotamase) activity and binds cyclosporin A. More preferred the activity of the protein is inhibited by cyclosporin A.

The protein can be used for *in vivo* and *in vitro* diagnostic and therapeutic means or remedies in form of solutions, bound to solid phases (i.e. to plates, beads, cups), dispersed or solved in cremes, powders, salves or analogous preparations. It can be employed as full length protein, in fragments, as epitopes, fused or conjugated to other proteins or substances or in clusters. The protein may be native or denaturated. The protein can be used alone or mixed with other purified allergens, or to crude extracts, for *in vivo* and *in vitro* diagnosis and treatment of allergic diseases.

Detailed description of isolation, identification and use of the protein of the present invention:

Allergen extraction: *Betula verrucosa* pollen were purchased from Allergon (Engelholm, Sweden). Extraction (1/10 w/v) was performed in phosphate-buffer(PB) 0.01M, pH 7.5 under continuous pH control and adjustment. After 3 h of extraction at room temperature, extracts were centrifuged (17500 g, 30 min) to remove pollen particles, and then filtrated through a 0.45-µm membrane.

Purification of allergens: The buffer of birch pollen extract was exchanged for Tris 0.05 M, pH 8.5. Extract was then incubated 3x15 min with each time fresh DEAE-Sephacel® (Pharmacia Biotech, Uppsala, Sweden) pre-equilibrated in Tris 0.05 M, pH 8.5, for a total gel/extract ratio of 3. After each incubation, the gel was removed by centrifugation (600 g, 5 min) and the supernatant was kept.

The resulting solution (fraction A) was dialyzed overnight against 1.4 M ammonium sulfate in 0.1 M di-sodium hydrogen phosphate, pH 7.0, before it was loaded into a phenylsepharose chromatography column (Pharmacia Biotech, Uppsala, Sweden) for hydrophobic interaction chromatography. Bound proteins were eluted by application of a 20-100% gradient of 0.1 M di-sodium hydrogen phosphate, pH 7.0. Fractions were collected and concentrated by membrane filtration (cut-off 10 kDa), and the buffer was exchanged for PB 0.01 M, pH 7.5, before the samples were aliquoted and frozen at -70°C until use.

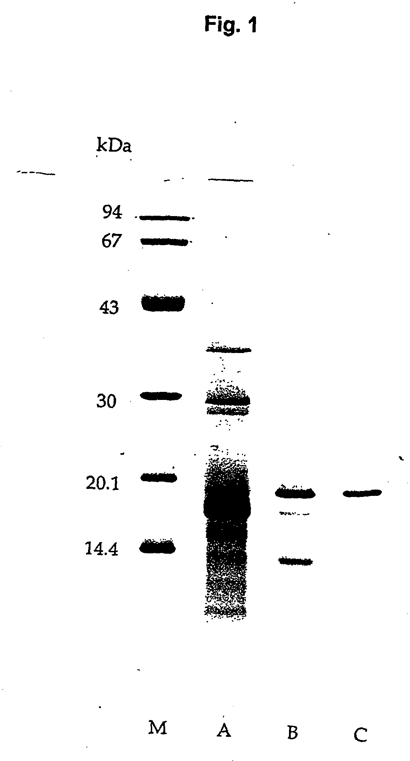

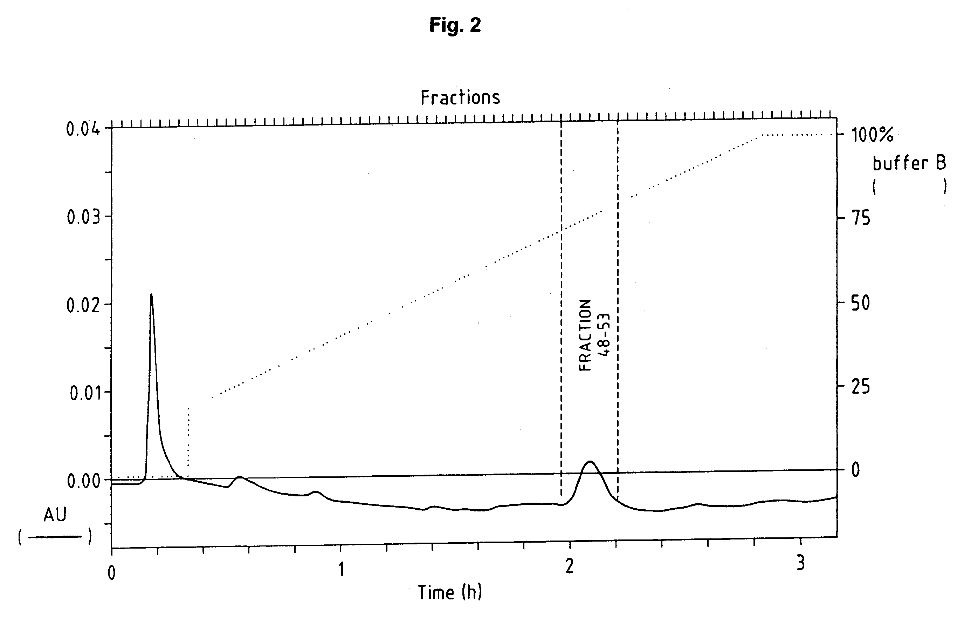

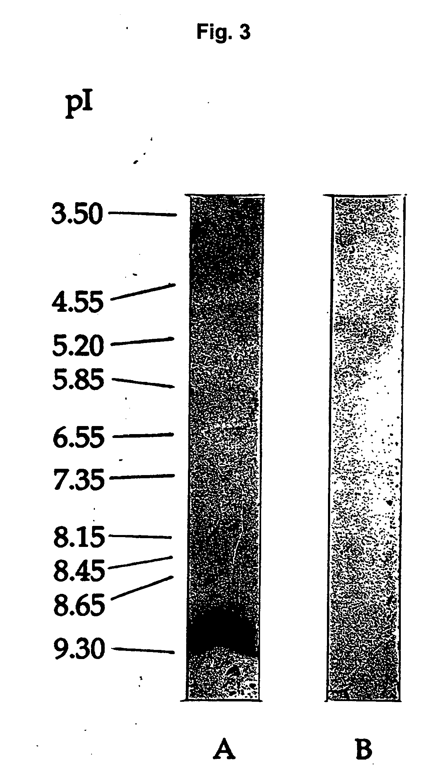

The steps of purification are illustrated in Fig.1. Incubation of crude extract with DEAE-Sephacel® at pH 8.5 yielded a solution enriched in 18-kDa protein (fraction A). The proteins of this fraction were further separated by hydrophobic interaction chromatography, for which a typical elution profile is shown in Fig.2. Screening of the resulting fractions was performed by IEF immunoblotting with human sera that were shown from Cadot *et al.* in Allergy 1995; 50: 431-437 to recognize the bands at pI 9.0, 9.1, and 9.3 in an IEF pattern. The target basic proteins were found in the fraction 48-53. This fraction gave a single band at 18-kDa in SDS-PAGE (Fig.1). A serum reacting with a high number of bands, including the basic ones, was used to probe IEF immunoblot strips of fraction 48-53. Only three bands at pI 9.0, 9.1, and 9.3 could then be distinguished (Fig.3).All three bound patients IgE. This confirmed that several isoforms of the cyclophilin must exist. Typically, amount of 250 pg of purified protein was obtained from 30 g of pollen. Concentration was determined by absorbance at 280 nm, assuming OD=0.6 for 1 mg/ml, which is the absorbance of tomato cyclophilin calculated from its whole sequence.

Electrophoresis and immunoblotting: Isoelectric focusing (IEF) was carried out using a Multiphor II device for horizontal electrophoresis (Pharmacia-Biotech, Uppsala,Sweden) in 5% thin-acrylamide gels containing 7.5% ampholytes with pH range 3-10 (Isolab, Akron, OH, USA) poured on Gelbond® PAG film (FMC, Rockland, ME, USA). The running conditions were 1500 V, 25 mA, and 15 W for a total of 1250 Vh. For immunoblotting experiments, proteins were transferred to PVDF membranes (Immobilon®, Millipore, Bedford, MA, USA) by press-blotting. After blocking in 0.2% nonfat dried milk in phosphate-buffered saline (PBS-NFDM 0.2%), membrane strips were incubated overnight at 4°C in patients sera diluted in PBS-NFDM 0.1% (125 µl of serum/strip). Bound IgE was afterwards detected by monoclonal mouse antihuman IgE antibodies (CLB, Amsterdam, The Netherlands), followed by peroxidase-conjugated goat antimouse IgG (CLB, Amsterdam, The Netherlands). Bands were revealed by incubation in the peroxidase substrate TMB (KPL, Gaithersburg, MD, USA).

SDS-PAGE was carried out in 13% polyacrylamide gels with 5% polyacrylamide stacking gel, using the discontinuous buffer system of Laemmli, Nature 1970, 227: 680-685. Before runs, samples were heated for 5 min at 100°C in sample buffer consisting of Tris 125 mM, pH 6.8, glycerol 17.5%, SDS 4%, beta-mercaptoethanol 1%, and bromophenol blue 0.002%. A voltage of 350 V was applied until the bromophenol blue reached the opposite side of the gel.

Microsequencing: The first attempts of N-terminal amino-acid sequencing with the purified protein were unsuccessful, which confirmed the N-terminal blockage which was already observed. The protein then was digested by Asp-N for 16 h at 37°C, and the resulting fragments separated by hydrophobic interactions in a HPLC column. Microsequencing was performed by Edman degradation in an automated sequencer.

Digestion with the protease Asp-N yielded several fragments. The analysis of one peak from the HPLC column gave clearly the following sequence: DFTAGNGTGGESIYGAK. The results of similarity searches with this sequence are presented in Table 1.

Similarity searches: Similarity searches and alignments of sequences were done in a computer by nonredundant searches using the Blitz server and the Swissall database.

The present invention refers to proteins, which include a sequence showing 100 % identity compared to SEQ ID N0:1.

**Table 1**

| Source | Molecular mass [Da] | Number of amino acids | Position of corresponding internal sequence | Identity with present cyclophilin sequence | accession number |
|---|---|---|---|---|---|
| Foxglove (*Digitalis lanata*) | 18055 | 172 | 73-89 | 100 % | Q96417 |
| French bean (*Phaseolus vulgaris*) | 18160 | 172 | 73-89 | 100 % | Q41119 |
| Rosy periwinkle (*Catharanthus roseus*) | 18285 | 172 | 73-89 | 100 % | Q39613 |
| Tomato (*Lycopersicon esculentum*) | 17910 | 171 | 73-89 | 100 % | P21568 |
| Onion (*Allium cepa*) | 16033 | 150 | 51-67 | 100 % | P34887 |
| Broad bean (*Vicia faba*) | ? | 171 | 73-89 | 94 % | D1026688 |
| Mouse-ear cress (*Arabidopsis thaliana*) | 18492 | 173 | 74-90 | 94 % | Q38900 |

Cyclophilin of the present invention shows a very great homology (up to 100% identity) with cyclophilins of various other plants. Plant cyclophilins, accordingly, have a molecular mass of about 18-kDa and a basic pI.

Rotamase assay: Rotamase activity was assayed by a modification of the original method of Fischer et al., Biochim. Biophys. Acta 1984; 791: 87-97. In this assay, a chromogenic peptide, the succinyl-Ala-Ala-Pro-Phe *p-*nitroanilide (sAAPFn; Sigma, St-Louis) is cleaved by chymotrypsin, but only when it is in the trans Ala-Pro conformation. More than 80% of the peptide is in this conformation at equilibrium. This fraction will be cleaved almost immediately by chymotrypsin, but the remaining 20% must undergo the relatively slow process of isomerization from cis to trans before it can be cleaved. Rotamase activity, that facilitates the cis-trans isomerization, then can be detected by the acceleration of the rate of peptide cleavage. In our assays, carried out in HEPES buffer 35 mM, pH 8.0, the final concentration of chymotrypsin was 2 µM, and that of sAAPFn was 10 µM. Rotamase activity was checked for purified protein concentrations of 3 nM and 30 nM. To inhibit rotamase activity, cyclosporin A (CsA) was used at 1 µM. As negative control for the inhibition of rotamase activity, 1 µM of FK506 was used, that is known to specifically bind another group of peptidyl-prolyl isomerases (rotamases), the FK506-binding proteins.

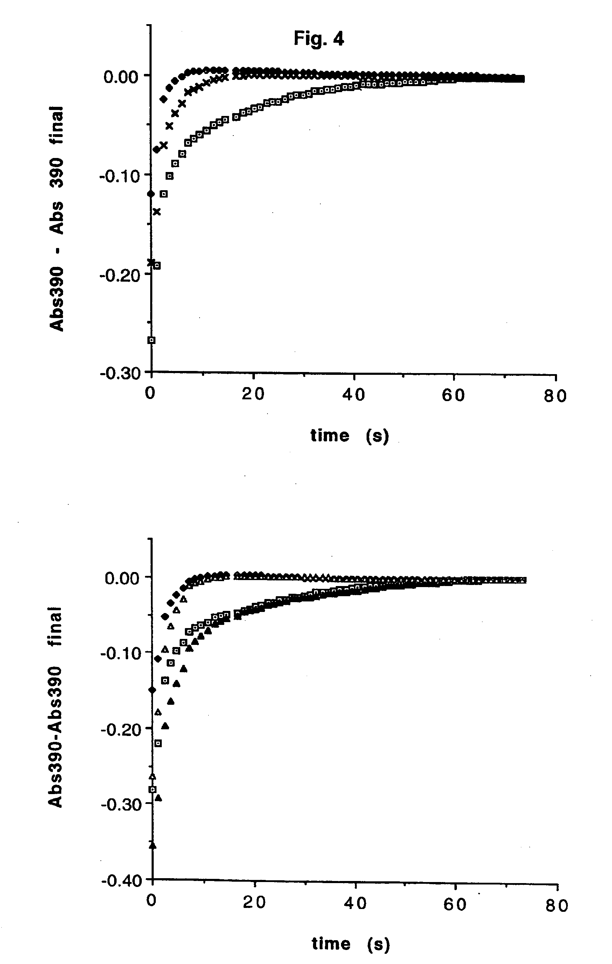

Rotamase activity has been demonstrated for cyclophilin in a dose-dependent manner (Fig.4). Furthermore, the allergen did bind CsA, which inhibited its rotamase activity (Fig.4), but did not bind FK506.

### Legends to the figures:

Fig. 1: Coomassie blue-stained SDS-PAGE gel showing: A, total birch pollen extract; B; fraction after DEAE-Sephacel; C, fraction 47-53 (4 µg) of hydrophobic interaction chromatography, Molecular mass markers are shown on the left.
Fig 2: Purification of birch pollen cyclophilin by phenylsepharose HP chromatography. Fraction A in buffer A was loaded into the column, and then eluted by a 20-100 % gradient of buffer B at a flow rate of 2 ml/min. Buffer A: 1.4 M (NH₄)₂SO₄; 0.1 M Na₂HPO₄; pH 7.0. Buffer B: 0.1 M Na₂HPO₄; pH 7.0. Absorbance (AU) was monitored at 280 nm.
Fig. 3: IgE-immunoblot from an IEF gel. The fraction 47-53 was probed with: A, the serum of a patient reacting to a high number of birch pollen allergens, including those in the basic range. B, incubation buffer as a control. pI markers are indicated on the left.
Fig. 4: Rotamase assays of purified birch pollen cyclophilin, involving: no cyclophilin (squares), 3 nM cyclophilin (crosses), 30 nM cyclophilin (black diamonds), 30 nM cyclophilin + 1 µM CsA (black triangles), 30 nM cyclophilin + 1 µM FK506 (open triangles). Absorbance at 390 nm reflects the cleavage of the chromogenic peptide sAAPFn.

### Example:

Skin testing: Skin prick tests with the fraction A (80 µg/ml in 50% glycerol) were performed on the forearm of seven birch pollen-allergic patients, selected by clear history and positive skin test to birch pollen. Thereafter, a test with the purified 18-kDa protein (at 1, 10, and 100 µg/ml in 50% glycerol) was carried out on patients who displayed a positive response to fraction A. A solution of 50% glycerol was used as negative control.

One out of the seven patients gave a clear positive response to the fraction A, and thereafter to the purified birch pollen cyclophilin (reaction very clear at 100 µg/ml). Control with 50% glycerol was negative. The sera of the seven patients skin tested were used in IEF immunoblots with total birch pollen extract There was correlation between the skin tests and the blots, since only the patient who was positive for the purified cyclophilin demonstrated binding to the three basic bands.

## Claims

1. Protein which is a plant cyclophilin containing SEQ ID NO: 1 for use in diagnosis and/or therapy of allergic diseases.

2. Protein of claim 1, wherein the plant is selected from tomato, foxglove, rosy periwinkle, onion or birch.

3. Protein of claim 1 or 2, wherein the plant cyclophilin is a birch pollen cyclophilin.

4. Use of the protein of any of claims 1 to 3, for the preparation of a means or remedy in diagnosis and/or therapy of allergic diseases.

5. Use of the protein according to claim 4, for the preparation of a means or remedy for in vivo and in vitro diagnosis or for therapy of pollen allergy and associated food-pollen cross-allergies.

6. Use according to claim 5, whereby the pollen allergy is selected from allergic rhinitis, allergic asthma and allergic conjunctivitis.

7. Use of the protein according to claim 4, for the preparation of a means or remedy in diagnosis and therapy of birch pollen allergy.

## Patentansprüche

1. Protein, welches ein Pflanzencyclophilin ist, das die SEQ ID NO: 1 enthält, zur Verwendung bei der Diagnose und/oder Therapie von allergischen Erkrankungen.

2. Protein nach Anspruch 1, wobei die Pflanze ausgewählt ist aus Tomate, Fingerhut, blühendem Immergrün, Zwiebel oder Birke.

3. Protein nach Anspruch 1 oder 2, wobei das Pflanzencyclophilin ein Birkenpollencyclophilin ist.

4. Verwendung des Proteins nach einem der Ansprüche 1 bis 3 für die Herstellung eines Hilfsmittels oder Heilmittels bei der Diagnose und/oder Therapie von allergischen Erkrankungen.

5. Verwendung des Proteins nach Anspruch 4 für die Herstellung eines Hilfsmittels oder Heilmittels für die in vivo und in vitro Diagnose oder für eine Therapie von Pollenallergie und damit in Zusammenhang stehenden Nahrungsmittel-Pollen-Kreuzallergien.

6. Verwendung nach Anspruch 5, wobei die Pollenallergie ausgewählt ist aus allergischer Rhinitis, allergischem Asthma und allergischer Konjunctivitis.

7. Verwendung des Proteins nach Anspruch 4 für die Herstellung eines Hilfsmittels oder Heilmittels für die Diagnose und die Therapie von Birkenpollenallergie.

## Revendications

1. Protéine qui est une cyclophiline végétale contenant SEQ ID N0: 1 pour une utilisation dans le diagnostic et/ou la thérapie des maladies allergiques.

2. Protéine selon la revendication 1, dans laquelle la plante est choisie parmi la tomate, la digitale pourprée, la petite pervenche rosée, l'oignon ou le bouleau.

3. Protéine selon la revendication 1 ou 2, dans laquelle la cyclophiline végétale est une cyclophiline de pollen de bouleau.

4. Utilisation de la protéine selon l'une quelconque des revendications 1 à 3, pour la préparation d'un moyen ou d'un remède dans le diagnostic et/ou la thérapie des maladies allergiques.

5. Utilisation de la protéine selon la revendication 4, pour la préparation d'un moyen ou d'un remède pour le diagnostic ou pour la thérapie in vivo et in vitro de l'allergie au pollen et des allergies croisées aliments et pollen associées.

6. Utilisation selon la revendication 5, moyennant quoi l'allergie au pollen est choisie parmi la rhinite allergique, l'asthme allergique et la conjonctivite allergique.

7. Utilisation de la protéine selon la revendication 4, pour la préparation d'un moyen ou d'un remède dans le diagnostic et la thérapie de l'allergie au pollen de bouleau.
